# EUROPÄISCHE PATENTSCHRIFT

(11) **EP 0 884 315 B1**
(45) Veröffentlichungstag und Bekanntmachung des Hinweises auf die Patenterteilung: **19.09.2001**
(21) Anmeldenummer: 98109716.5
(22) Anmeldetag: 28.05.1998
(51) Int. Cl.: C07D 321/00, C11B 9/00

(54) **Dioxacycloalkan-8-one**
Dioxacycloalkan-8-ones
Dioxacycloalkan-8-ones

(30) Priorität: 09.06.1997 EP 97109303
(43) Veröffentlichungstag der Anmeldung: 16.12.1998
(73) Patentinhaber: Givaudan SA, 1214 Vernier-Genève (CH)
(72) Erfinder: Kraft, Philip, 8600 Dübendorf (CH)
(74) Vertreter: Patentanwälte Schaad, Balass, Menzl & Partner AG

(56) Entgegenhaltungen:
- EP-A- 0 023 612
- EP-A- 0 093 852
- EP-A- 0 103 893
- US-A- 2 202 448
- US-A- 2 234 551

## Beschreibung

Die Erfindung betrifft 14- bis 17-gliedrige 4-methylsubstituierte 1,7-Dioxacycloalkan-8-one und 14- bis 16-gliedrige 4,4-dimethylsubstituierte 1,7-Dioxacycloalkan-8-one, die Verwendung dieser Verbindungen als Riechstoffe, sowie ein kostengünstiges Verfahren zu ihrer Herstellung.

*Warme, süss-pudrige Basisnoten* mit moschusartigen Geruchseigenschaften, die von *blumig-süssen* (Ambrett Moschus) über *pudrig-animalische* (Moschus Keton) bis sogar hin zu *herb-holzigen Tonalitäten* (Moschus Xylol) reichen können, sind nahezu unverzichtbar bei der Komposition eines Parfüms, ebenso wie bei der Parfümierung z.B. von Kosmetika, Wasch- und Reinigungsmitteln, Weichspülern oder Luftverbesserern. Aufgrund solcher Geruchscharakteristika und nicht zuletzt auch wegen ihrer technisch einfachen und preiswerten Synthese gewannen aromatische Moschuskörper besondere Bedeutung in der Parfümerie und wurden universell und in hoher Dosierung eingesetzt. Diese Verbindungen, insbesondere Moschus Xylol und Ambrett Moschus, besitzen jedoch eine gewisse Toxizität, insbesondere Phototoxizität, und schlechte biologische Abbaubarkeit. Je nach territorialer Regelung sollten oder dürfen sie in neuen Kreationen nicht mehr verwendet werden und sollten oder müssen auch in älteren durch andere Verbindungen ersetzt werden.

Andere Verbindungen mit moschusartigen Geruchseigenschaften, insbesondere toxikologisch unbedenkliche Makrocyclen, sind zum einen jedoch deutlich teurer und besitzen zum anderen aber auch andere Nebennoten. Daher ändert sich bei Ersatz eines Nitromoschuses durch einen Makrocyclus der Gesamtgeruchseindruck einer Komposition meist stark. Ausserdem sind die zur Zeit kommerziell erhältlichen Makrocyclen bei weitem nicht so facettenreich wie die Reihe obgenannter hochsubstituierter Aromaten. Aus all dem erhellt, dass ein grosser Bedarf an neuen toxikologisch unbedenklichen makrocyclischen Verbindungen mit neuen Geruchsnoten, insbesondere solchen, die an aromatische Moschuskörper erinnern oder mit denen sich ähnlich facettenreiche parfümistische Effekte erzielen lassen, besteht. Darüber hinaus sollten sich derartige makrocyclische Verbindungen einfach und preiswert herstellen lassen, um nicht nur in Luxusparfüms, sondern auch in Produkten des täglichen Haushaltsbedarfs, z.B. in Kosmetika, Wasch- und Reinigungsmitteln, Weichspülern oder Luftverbessern, eingesetzt werden zu können.

Die Aufgabe der vorliegenden Erfindung besteht nun darin, diesem Bedarf entgegenzukommen und derartige Verbindungen zur Verfügung zustellen. Diese Verbindungen sollten sich darüber hinaus noch kostengünstig herstellen lassen.

Die Aufgabe wird durch die Verbindungsklasse der 4-methylsubstituierten 1,7-Dioxacycloalkan-8-one gelöst, die den geforderten Ansprüchen in idealer Weise genügt. Sie wird durch die allgemeine Formel **I** wiedergegeben, 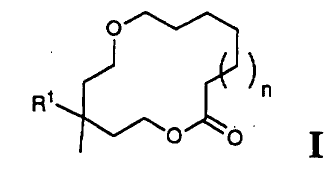 worin R¹ = H bei n = 1-4
oder R¹ = CH₃ bei n = 1-3 darstellt.

Die erfindungsgemässe Verbindungsklasse umfasst demnach die 14- bis 17-gliedrige 1,7-Dioxacycloalkan-8-one mit Methyl- und die 14- bis 16-gliedrigen 1,7-Dioxacycloalkan-8-one mit Dimethylsubstitution in 4-Position. Die Verbindungen mit den folgenden Formeln **1-6** sind exemplarisch für die neue Verbindungsklasse, wobei sich die Verbindungen mit den Formeln 1, 3 und 4 organoleptisch besonders auszeichnen.

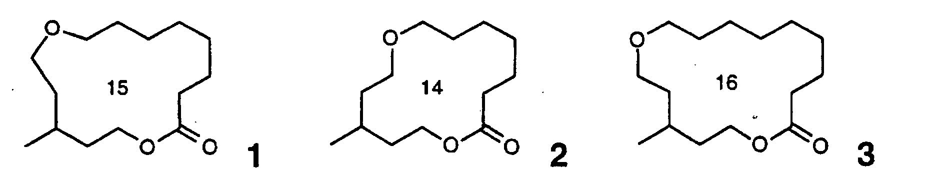

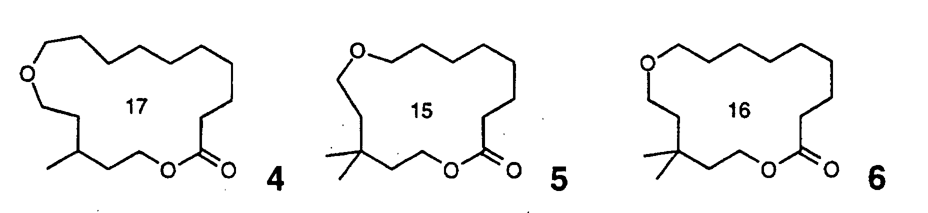

Die Verbindungen der allgemeinen Formel **I** besitzen parfümistisch interessante, *pudrig- warme, grösstenteils moschusartige Geruchsnoten mit frisch-floralen bis kräuterartigen Akzenten* sowie eine guten Haftfestigkeit und sind biologisch abbaubar. Sie sind in der Lage, allein oder in Verbindung mit anderen makrocyclischen Riechstoffen toxikologisch bedenkliche Verbindungen mit gleichen oder ähnlichen Geruchsnoten wie etwa Ambrett Moschus in den eingangs genannten Anwendungsbereichen zu ersetzen. Sie ergeben ferner aufgrund ihrer facettenreichen Geruchseigenschaften sowohl einzeln als auch in Kombination bei Neukompositionen interessante neue Effekte. Die Geruchsnoten reichen dabei von den 14 - zu den 17-gliedrigen Verbindungen von *schwach moschusartig, anisig, krautig, Safran, Myrrhe zu stark moschusartig, animalisch.* Insbesondere die Verbindungen **1, 3** und **4** sind diesbezüglich besonders markant.

Die Verbindungen der allgemeinen Formel **I** harmonieren mit einer Vielzahl natürlicher und synthetischer Produkte, die häufig in Riechstoffkompositionen eingesetzt werden. Besonders in der Duftrichtung Chypre, zum Beispiel in Kombination mit einem ledrigen Akkord, oder in floralen Salicylat-Parfüms ergeben die Verbindungen der allgemeinen Formel **I** sehr interessante parfümistische Effekte. Ideal eignet sich insbesondere die Verbindung **1** aber auch für die Komposition von süssen Fougère-Noten vom Typ "Brut" (Fabergè, 1964) und seinen komplexeren modernen Nachfolgern. Der Einsatz ist jedoch weder auf diese Parfümtypen noch auf spezielle Duftrichtungen, Riechstoffe oder Substanzklassen beschränkt. Beispiele für besonders gut harmonierende Stoffklassen sind:
- - Ätherische Öle und Extrakte:: z. B. Bibergeil, Costuswurzelöl, Eichenmoos Absolue, Geraniumöl, Jasmin Absolue, Patchouliöl, Rosenöl, Sandelholzöl oder Ylang-Ylang-Öl;
- - Alkohole:: z. B. Citronellol, Ebanol® , Eugenol, Geraniol, Javanol® , Linalool, Phenylethylalkohol, Sandalore® , Terpineol oder Timberol® ;
- - Aldehyde und Ketone:: z. B. *alpha*-Amylzimtaldehyd, Georgywood®, Hydroxycitronellal, Iso-E-Super® , Isoraldein® , Hedion® , Maltol, Methylcedrylketon, Methyljonon oder Vanillin;
- - Ether und Acetale:: z. B. Ambrox® , Geranylmethylether, Rosenoxid oder Spirambrene® ;
- - Ester und Lactone:: z. B. Benzylacetat, Cedrylactetat, γ-Decalacton, γ-Undecalacton oder Vetiverylacetat;
- - Makrocyclen:: z. B. Ambrettolid, Musk TM II® oder Exaltolid® ;
- - Heterocyclen:: z. B. Isobutylchinolin.

Die Aufgabe der kostengünstigen Synthese der Verbindungen der allgemeinen Formel **I** wird erfindungsgemäss durch einen neuen Zugang gelöst, der von aliphatischen Dihalogeniden und Diolen, welche gut zugängliche und preisgünstige Ausgangsmaterialien sind, ausgeht. Die Synthesesequenz basiert auf einer neuen Eintopfreaktion aus Decarboxylierung und Cyclisierung, die es ermöglicht, aliphatische Hydroxymalonester direkt zu 14- bis 17-gliedrigen Makroliden zu cyclisieren.

Die neue Synthesesequenz zu 14- bis 17-gliedrigen Oxamakroliden, über die insbesondere die Verbindungen der allgemeinen Formel **I** preisgünstig zugänglich werden, beginnt, wie nachfolgend schematisch dargestellt ist, mit der Williamson Reaktion von einem aliphatischen Dihalogenid **7** mit n = **1 - 4** und X=Br oder Cl und einem Diol **8** , wobei für die Synthese der Verbindungen der allgemeinen Formel **I**
R¹ = H oder Me ist, in Anwesenheit einer starken Base, z.B. von Natriumhydrid. Neben dem dimeren Kondensationsprodukt **9** erhält man dabei auch höhere Oligomere, die jedoch die weitere Reaktion nicht stören, so dass das Zwischenprodukt **9** nicht aufgereinigt werden muss. Vorzugsweise werden unverbrauchte Ausgangsverbindungen **7** und **8** nach der Reaktion zur Wiederverwendung destillativ abgetrennt.

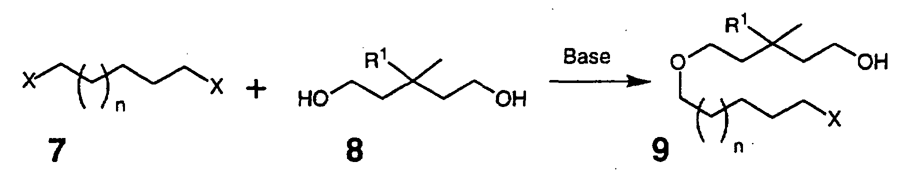

Als nächster Schritt der Sequenz wird in an sich bekannter Weise eine Malonesterkondensation durchgeführt, die aus dem Halogenalkohol 9 den Hydroxymalonester 10 liefert, wobei R² = Alkyl, vorzugsweise Me oder Et, ist.

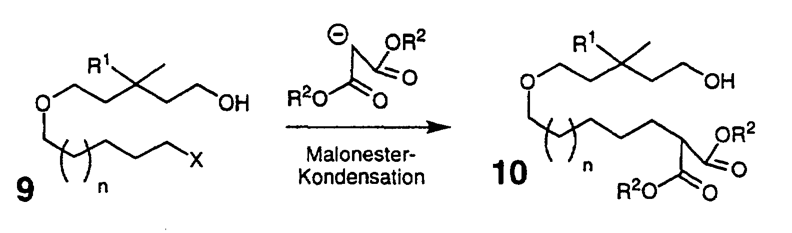

Erfindungsgemäss wird der Hydroxymalonester 10 nun ohne Zwischenreinigung in der neuen Eintopfreaktion verseift und unter Decarboxylierung polymerisiert. Die Depolymerisierung und Cyclisierung zur Zielverbindung, insbesondere zu einer Verbindung der allgemeinen Formel I, erfolgt durch an sich bekannte Verfahren, z.B. nach dem Verfahren, wie es im U.S. Patent 2234551 beschrieben ist, was schematisch nachfolgend dargestellt ist.

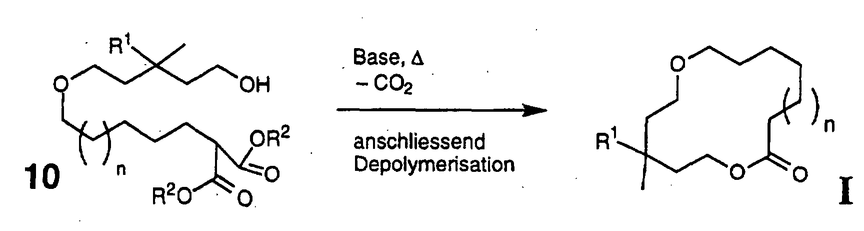

Durch die Eintopf-Decarboxylierung-Polymerisation und durch die beschriebene Synthesesequenz wird die Verwendung teurer langkettiger aliphatischer Halogencarbonsäuren, die den Hauptkostenfaktor bei der herkömmlichen Synthese von 14-bis 17-gliedrigen Oxamakroliden darstellen, umgangen.
Obwohl die beschriebene Synthesesequenz bevorzugt auf die Herstellung von Verbindung der allgemeinen Formel **I** ausgerichtet ist, ist sie auch erfindungsgemäss für die kostengünstige Herstellung bereits bekannter 14- bis 17-gliedriger Oxamakrolide anwendbar.

Die erfindungsgemässen Verbindungen, vorzugsweise die Verbindungen **1** bis **6**, besonders bevorzugt die Verbindungen **1**, **3** und **4**, insbesondere die Verbindung **1**, können einzeln oder in Kombination als Riechstoff verwendet werden. Wenn in einer Riechstoffkombination die jeweilige Verbindung mit einem Inhalt von etwa 0.1 bis etwa 25 Gew.-%, vorzugsweise von 10 bis 15 Gew.-%, vertreten ist, werden besonders interessante olfaktorische Effekte erzielt.

Weitere Vorteile, Merkmale und Einzelheiten zur Erläuterung der Erfindung ergeben sich aus der nachfolgenden Beschreibung bevorzugter Ausführungsbeispiele:

### Beispiel 1: Herstellung von 12-Methyl-9-oxa-14-tetradecanolid (1)

118 g (1.0 mol) 3-Methyl-1,5-pentandiol und 317 g (1.3 mol) 1,6-Dibromhexan in 2.5 1 Dioxan wurden bei Raumtemperatur mit 28 g (1.1 mol) 95 prozentigem Natriumhydrid versetzt und anschliessend 24 h unter Rückfluss erhitzt. Nach dem Abkühlen wurde das Reaktionsgemisch mit 400 ml Wasser versetzt, mit gesättigter Ammoniumchlodrid-lösung neutralisiert und anschliessend dreimal mit je 1 1 *tert*-Butylmethylether extrahiert. Die vereinigten organischen Extrakt wurden mit gesättigter Ammoniumchlodridlösung gewaschen, über Magnesiumsulfat getrocknet und am Rotationsverdampfer eingeengt. Ausgangsmaterial und Nebenprodukte wurden durch Destillation in Vakum bei 150 °C Badtemperatur und 0.09 mbar entfernt. Als Destillationsrückstand erhielt man 183 g 66 prozentigen 12-Brom-3-methyl-6-oxadodecan-1-ol (43%), was für die weiteren Umsetzungen ausreichend rein ist.

Eine Probe, durch Kugelrohrdestillation bei 200 °C / 0.03 mbar gereinigt, zeigt die folgenden spektroskopischen Daten:

IR (Film): ν = 1112 cm⁻¹ (νₐₛ C-O-C), 1060 cm⁻¹ (νₛ C-O-C), 3389 cm⁻¹ (ν O-H), 1459 cm⁻¹ (δ C-H), 1377 cm⁻¹ (δ CH₃). - ¹H-NMR (CDCl₃): δ = 0.93 (d, *J* = 6.8 Hz, 3H, 3-Me), 1.35-1.49 (m, 6H, 2-, 9-,10-H₂), 1.55-1.65 (m, 4H, 4-,8-H₂), 1.72 (oct, *J=* 6.8 Hz, 1H, 3-H), 1.89 (quint, *J* = 6.8 Hz, 2H, 11-H₂), 2.19 (br s, 1H, OH), 3.39-3.49 (m, 6H, 5-,7-,12-H₂), 3.65 (ddd, *J* = 10.4, 4.0 und 4.0 Hz, 1H, 1-H_{b}), 3.71 (ddd, *J* = 10.4, 3.7 und 3.7, 1H, 1-Hₐ). - ¹³C-NMR (CDCl₃): δ = 19.86 (q, 3-Me), 25.30 (t, C-9), 26.76 (d, C-3), 27.89 (t, C-10), 29.42 (t, C-8), 32.63 (t, C-12), 33.84 (t, C-11 ), 36.43 (t, C-4), 39.69 (t, C-2), 60.73 (t, C-1), 68.96 (t, C-5), 70.72 (t, C-7). - MS (EI): m/z (%) = 83 (100) [C₆H₁₁^{⊕}], 99 (61) [C₆H₁₁O^{⊕}], 117 (7), [M^{⊕}-C₆H₁₂Br], 163/165 (6) [M^{⊕} -C₆H₁₃O₂], 263/265 (2) [M^{⊕} -OH].

Eine Lösung von 30 g (520 mmol) 95 prozentiges Natriummethylat in 220 ml trockenem Methanol wurde zum Rückfluss erhitzt. Darauf liess man 60 ml (520 mmol) Malonsäuredimethylester zutropfen, erhitzte die Reaktionsmischung weitere 15 min unter Rückfluss und gab dann 182 g 66 prozentigen 12-Brom-3-methyl-6-oxadodecan-1-ol aus vorangehendem Ansatz zu. Nach 14 stündigem Erhitzen unter Rückfluss wurde das Reaktionsgemisch auf 1.6l Wasser/*tert*-Butylmethylether (1:1) gegeben und mit konzentrierter Phosphorsäure sauer gestellt. Die organische Phase wurde abgetrennt, die wässrige Phase zweimal mit je 500 ml *tert*-Butylmethylether extrahiert. Die vereinigten organischen Phasen wurden über Magnesiumsulfat getrocknet, am Rotationsverdampfer zur Trockne eingeengt, in 400 ml Methanol aufgenommen und mit 99 g (1.5 mol) 85 prozentigem Kaliumhydroxid versetzt. Bei 180 °C 20-23 mbar wurden während 1 h Methanol und Nebenprodukte abdestilliert. Darauf wurde das Reaktionsgemisch mit 200 ml (2.4 mol) 3-Chlor-1,2-propandiol versetzt und 1 h auf 140 °C erhitzt. Nach Abdestillieren des überschüssigen 3-Chlor-1,2-propandiols wurde das Reaktionsgefäss mit Kühler und Abscheider ausgerüstet und mit 6.0 g (86 mmol) Kaliummethylat in 450 ml wasserfreiem Glycerin versetzt. Nach 15 minütigem Rühren bei 140°C / 20-30 mbar erhitzte man bei 155°C / 4-6 mbar 3 Tage zum Rückfluss über den Abscheider, wobei jeweils nach 24 Stunden erneut 6.0 g (86 mmol) Kaliummethylat zugegeben wurden. Das abgeschiedene Glycerin wurde auf 800 ml Wasser gegossen und dreimal mit je 500 ml *tert*-Butylmethylether extrahiert. Nach Trocknen der vereinigten organischen Extrakte über Magnesiumsulfat, Einengen am Rotationsverdampfer und Destillation bei 95-97 °C / 0.04 mbar erhielt man 59 g (52%) 12-Methyl-9-oxa-14-tetradecanolid (1) als farblose Flüssigkeit mit folgenden Charakteristika.

Geruch: *Moschus, blumig-holzig, süss-pudrig, leicht anisartig, frisch, nach Myrrhe an Ambrett Moschus, Moschuskörneröl und Tonkinmoschus erinnernd.* - IR (Film): v = 1735 cm⁻¹ (ν C=O), 1116/ 1153 cm⁻¹ (νₐₛ O-C-C), 1247 cm⁻¹ (νₐₛ C-C(=O)-O), 1209 cm⁻¹ (νₐₛ C-O-C), 1352 cm⁻¹ (δ CH3), 1054 cm⁻¹ (νₐₛ C-O-C). - ¹H-NMR (CDCl₃): δ = 0.94 (d, *J =* 6.4 Hz, 3H, 12-Me), 1.27-1.76 (m, 14H, 3-H₂-7-H₂ und 11-,13-H2), 1.89 (oct, *J* = 6.7 Hz, 1H, 12-H), 2.34 (t, *J* = 6.4 Hz, 2H, 2-H₂), 3.37-3.52 (m, 4H, 8-,10-H₂), 4.16 (ddd, *J =* 11.2, 9.9 und 3.3 Hz, 1H, 14-H_{b}), 4.19 (ddd, *J* = 11.2, 5.4 und 5.2 Hz, 1H, 14-Hₐ). - ¹³C-NMR (CDCl₃): δ= 19.22 (q, 12-Me), 24.46 (t, C-3), 24.95 (d, C-12), 25.92 (t, C-6), 27.02/28.12/ 28.44 (t, C-4,-5,-7), 33.89 (t, C-2), 35.45 / 36.62 (t, C-11,-13), 61.51 (t, C-14), 67.60/69.61 (t, C-8,-10), 173.91 (s, C-1). - MS (EI): m/z (%) = 55 (100) [C₄H₇^{⊕} ], 83 (59) [C₆H₁₁^{⊕}], 99 (24) [C₆H₁₁O^{⊕}], 124 (31) [M^{⊕}-H₂O-C₆H₁₂O], 141 (19) [M^{⊕}-H₂O-C₆H₁₁], 213 (1) [M^{⊕}-CHO], 242 (1) [M^{⊕}].-C₁₄H₂₆O₃ (242.36): berechnet C 69.38, H 10.81; gefunden C 69.24, H 10.68.

Die folgenden Verbindungen sind in analoger Weise unter Verwendung unterschiedlich langer Dihalogenide 6 hergestellt worden. Von ihnen sind daher nur die spektroskopischen Daten, die Geruchscharakteristiken und die Elementaranalysen aufgeführt:

### Beispiel 2: 11-Methyl-8-oxa-13-tridecanolid (2)

Geruch: *Safran, anisartig, holzig-blumig, kienig-terpenig, frisch, schwach moschusartig.* -IR (Film): ν = 1734 cm⁻¹ (ν C=O), 1123 / 1155 cm⁻¹ (νₐₛ O-C-C), 1254 cm⁻¹ (νₐₛ C-C(=O)-O), 1203 cm⁻¹ (νₐₛ C-O-C), 1357 cm⁻¹ (δ CH₃), 1056 cm⁻¹ (νₐₛ C-O-C). - ¹H-NMR (CDCl₃): δ=0.96 (d, *J* = 6.8 Hz, 3H, 11-Me), 1.15 (dddd, *J* = 14.5, 10.0, 4.8 und 2.4 Hz, 1H, 12-H_{b}), 1.25-1.79 (m, 10H, 3-H₂-6-H₂,10-H₂), 1.85 (dddd, *J* = 14.5, 7.6, 7.3 und 3.9 Hz, 1H, 12-Hₐ), 1.99 (m_{c}, 1H, 11-H), 2.31 (ddd, *J* = 14.8, 8.3 und 3.7 Hz, 1H, 2-Hb), 2.41 (ddd, *J* = 14.8, 9.3 und 3.7 Hz, 1H, 2-Hₐ), 3.33-3.50 (m, 4H, 7-,9-H₂), 3.98 (ddd, *J* = 11.2, 11.0 und 2.4 Hz, 1H, 13-H_{b}), 4.53 (ddd, *J* = 11.2, 4.6 und 3.6 Hz, 1H, 13-Hₐ).-¹³C-NMR (CDCl₃): δ = 19.47 (q, 11-Me), 23.72 (d, C-11), 25.32/25.51/26.81 (t, C-3-C-5), 28.62 (t, C-6), 33.79 (t, C-2), 34.53 (t, C-12), 36.51 (t, C-10), 60.96 (t, C-13), 66.91 / 68.85 (t, C-7,-9), 173.78 (s, C-1).- MS (EI): m/z (%) = 55 (100) [C₄H₇^{⊕}], 83 (79) [C₆H₁₁^{⊕}], 101 (48) [C₆H₁₃O^{⊕}], 111 (24) [C₆H₁₃O^{⊕}], 127 (30) [M^{⊕}-C₆H₁₃O], 145 (4) [M^{⊕}-C₆H₁₁], 169 (2) [M^{⊕}-C₂H₃O₂], 199 (1) [M^{⊕} -CHO], 228 (1) [M^{⊕}]. - C₁₃H₂₄O₃ (228.33): berechnet C 68.38, H 10.60; gefunden C 68.55, H 10.57.

### Beispiel 3: 13-Methyl-10-oxa-15-pentadecanolid (3)

Geruch: *Moschus, animalisch, warm-pudrig, blumig, schwach nach Safran. -* IR (Film): ν = 1734 cm⁻¹ (ν C=O), 1118/1151 cm⁻¹ (νₐₛ O-C-C), 1250 cm⁻¹ (νₐₛ C-C(=O)-O), 1357 cm⁻¹ (δ CH₃), 1061 cm⁻¹ (νₐₛ C-O-C). - ¹H-NMR (CDCl₃): δ = 0.91 (d, *J* = 6.4 Hz, 3H, 13-Me), 1.31-1.66 (m, 15H, 3-H₂-8-H₂,12-H₂, 14-H_{b}), 1.83 (ddt, *J =* 19.1, 9.6 und 5.0 Hz, 1H, 14-Hₐ), 1.93 (m_{c}, 1H, 13-H), 2.33 (dd, *J* = 6.9 und 6.2 Hz, 2H, 2-H₂), 3.34-3.52 (m, 4H, 9-,11-H₂), 4.14 (ddd, *J =* 11.0, 5.5 und 5.0 Hz, 1H, 15-H_{b}), 4.21 (ddd, *J* = 11.0, 10.0 und 4.0 Hz, 1H, 15-Hₐ). - ¹³C-NMR (CDCl₃): δ = 18.22 (q, 13-Me), 24.67/25.26 (t, C-3,-7), 25.53 (d, C-13), 27.21 / 27.40 / 27.52 (t, C-4,-5,-6), 28.68 (t, C-8), 34.67 (t, C-2), 35.49 (t, C-14), 37.01 (t, C-12), 61.74 (t, C-15), 67.67 / 70.16 (t, C-9,-11), 173.93 (s, C-1). - MS (EI): m/z (%) = 55 (100) [C₄H₇^{⊕}], 83 (64) [C₆H₁₁^{⊕}], 99 (30) [C₆H₁₁O^{⊕}], 138 (25) [M^{⊕}-C₆H₁₄O₂], 155 (23) [M^{⊕}-C₆H₁₃O], 213 (2) [M^{⊕}-C₂H₃O], 227 (2) [M^{⊕}-CHO], 256 (1) [M^{⊕}]. -C₁₅H₂₈O₃ (256,39): berechnet C 70.27, H 11.01; gefunden C 70.26, H 11.09.

### Beispiel 4: 14-Methyl-11-oxa-16-hexadecanolid (4)

Geruch: *Animalisch, Moschus, süss, erogen, warm-pudrig. -* IR (Film): v = 1735 cm⁻¹ (ν C=O), 1117/1151 cm⁻¹ (νₐₛ O-C-C), 1254 cm⁻¹ (νₐₛ C-C(=O)-O), 1361 cm⁻¹ (δ CH₃), 1060 cm⁻¹ (νₐₛ C-O-C). - ¹H-NMR (CDCl₃): δ=0.92 (d, *J =* 6.8 Hz, 3H, 14-Me), 1.29-1.56 (m, 14H, 4-H₂-9-H₂, 13-H₂), 1.61-1.67 (m, 3H, 3-H₂, 15-H_{b}), 1.79 (ddt, 14.0, 8.4 und 5.2 Hz, 1H, 15-Hₐ), 1.87 (m_{c}, 1H, 14-H), 2.33 (dd, *J* = 6.8 und 6.0 Hz, 2H, 2-H), 3.34-3.50 (m, 4H, 10-,12-H₂), 4.15 (ddd, *J* = 11.2, 6.0 und 5.2 Hz, 1H, 16-H_{b}), 4.18 (ddd, *J* = 11.2, 8.4 und 4.4 Hz, 1H, 16Hₐ). - ¹³C-NMR (CDCl₃): δ = 18.54 (q, 14-Me), 24.80 / 26.15 (t, C-3,-8), 26.17 (d, C-14), 27.31/27.66/27.79/28.22 (t, C-4-C-7), 29.15 (t, C-9), 34.02 (t, C-2), 35.57 (t, C-15), 37.00 (t, C-13), 62.14 (t, C-16), 68.05/70.77 (t, C-10,-12), 174.14 (s, C-1). -MS (EI): m/z (%) = 55 (100) [C₄H₇^{⊕}], 83 (71) [C₆H₁₁^{⊕}], 99 (33) [C₆H₁₁O^{⊕}], 113 (13) [M^{⊕}-C₁₀H₂₁O], 153(15) [M^{⊕} -C₆H₁₃O₂], 169(16) [M^{⊕}-C₆H₁₃O], 171 (9) [C₁₀H₁₉O₂^{⊕}], 227 (1) [M^{⊕}-C₂H₃O], 241 (2) [M^{⊕}-CHO], 270 (1) [M^{⊕}].- C₁₆H₃₀O₃ (270.41): berechnet C 71.07, H 11.18; gefunden C 71.37, H 11.13.

### Beispiel 5: 12,12-Dimethyl-9-oxa-14-tetradecanolid (5)

Geruch: *relativ schwach, pudrig -moschusartig, holzig-krautig.-* IR (Film): v = 1734 cm-1 (ν C=O), 1117/1154 cm⁻¹ (νₐₛ O-C-C), 1252 cm⁻¹ (νₐₛ C-C(=O)-O), 1366 cm⁻¹ (δ CH₃), 1046 cm⁻¹ (νₐₛ C-O-C). - ¹H-NMR (CDCl₃): δ = 0.94 (s, 6H, 12-Me₂), 1.31-1.42 (m, 6H, 4-H₂-6-H₂), 1.50-1.57 (m, 4H, 3-,7-H₂), 1.69 (t, *J* = 7.2 Hz, 2H, 11-H₂), 1.71 (dd, *J* = 10.0 und 6.8 Hz, 2H, 13-H₂), 2.30 (t, *J* = 6.6 Hz, 2-H₂), 3.39 (t, *J =* 5.2 *Hz,* 8-H₂), 3.46 (t, *J* = 6.2 Hz, 10-H₂), 4.16 (t, *J* = 7.0 Hz, 2H, 14-H₂). - ¹³C-NMR (CDCl₃): δ = 23.72, 24.91 (t, C-3,-6), 26.41/27.89 (t, C-4,-5), 28.59 (2q, 12-Me₂), 29.05 (t, C-7), 31.77 (s, C-12), 34.59 (t, C-2), 38.86/40.57 (t, C-11,-13), 61.93 (t, C-14), 67.66/69.88 (t, C-8,-10), 174.15 (s, C-1).-MS (EI): m/z (%) = 55 (100) [C₄H₇^{⊕}], 69 (72) [C₅H₉^{⊕}], 81 (40) [C₆H₉^{⊕}], 97 (59) [C₆H₉O^{⊕}], 113 (27) [C₇H₁₃O^{⊕}], 125 (27) [C₈H₁₃O^{⊕}], 141 (15) [M^{⊕}-C₆H₉O-H₂O], 183 (2) [M^{⊕}-C₄H₇-H₂O], 227 (1) [M^{⊕}-CHO], 256 (1) [M^{⊕} ]. -C₁₅H₂₈O₃ (256,39): berechnet C 70.27, H 11.01; gefunden C 70.15, H 10.87.

### Beispiel 6: 13,13-Dimethyl-10-oxa-15-pentadecanolid (6)

Geruch: *relativ schwach, fruchtig-moschusartig, an Ambretton erinnernd. -* IR *(Film):* ν = 1734 cm⁻¹ (ν C=O), 1118 / 1150 cm⁻¹ (νₐₛ O-C-C), 1242 cm⁻¹ (νₐₛ C-C(=O)-O), 1365 cm⁻¹ (δ CH₃), 1055 cm⁻¹ (νₐₛ C-O-C). - ¹H-NMR (CDCl₃): δ = 0.95 (s, 6H, 13-Me₂), 1.33-1.40 (m, 8H, 4-H₂-7-H₂), 1.49-1.55 (m, 4H, 8-,12-H₂), 1.62 (m_{c}, 2H, 3-H₂), 1.76 (t, *J* = 8.0 Hz, 2H, 14-H₂), 2.32 (t, *J* = 6.4 Hz, 2H, 2-H₂), 3.38 (t, *J* = 5.2 Hz, 2H, 11-H₂), 3.47 (t, *J* = 5.8 Hz, 2H, 9-H₂), 4.16 (t, *J* = 8.0 Hz, 2H, 15-H₂). - ¹³C-NMR (CDCl₃): δ = 23.94 / 24.56 (t, C-3,-7), 25.84 / 26.36 / 26.52 (t, C-4,-5,-6), 28.04 (2q, 13-Me₂), 29.23 (t, C-8), 31.79 (s, C-13), 34.53 (t, C-2), 39.02 (t, C-14), 41.28 (t, C-12), 61.98 (t, C-15), 67.82 / 70.49 (t, C-9,-11), 173.83 (s, C-1). - MS (EI): m/z (%) = 55 (100) [C₄H₇^{⊕}], 69 (92) [C₅H₉^{⊕}], 113 (39) [C₈H₁₇^{⊕}], 139 (29) [M^{⊕}-C₇H₁₅O₂], 155 (18) [M^{⊕} -C₇H₁₅O], 197 (3) [M^{⊕}-CO-C₂H₄O], 241 (2) [M^{⊕}-CO], 270 (1) [M^{⊕} ]. - C₁₆H₃₀O₃ (270.41): berechnet C 71.07, H 11.18; gefunden C 70.89, H 11.11.

### Beispiel 7:

Es wurde eine Herren-Parfümkomposition "Oriental Fougere" hergestellt. Die Komponenten sind unten aufgeführt. Dazu wurden 15-Gew.-% der Verbindung 1 anstatt des für diesen Parfümkompositionstyp üblichen Moschus Ambrett verwendet. Die Komposition enthält keine polycyclischen Moschusriechstoffe.

Die Verbindung **1** verleiht dem Parfüm eine Moschusnote, bringt süsslich-pudrige, an Nitromoschus erinnernde Effekte ein und ist somit mehr als ein Substitut für Nitromoschus. Ihre Pudrigkeit zusammen mit dem Tonkinmoschusaspekt unterstreichen den warm-pudrigen Geruchseindruck und tragen zur Abrundung der Kopfnote und der Gesamtkomposition bei.

### Zusammensetzung:

| Ingredienzien | Gewichts %o |
|---|---|
| 1. Aldehyd C11 (10-Undecen-1-al) | 0.05 |
| 2. Aldehyd C12 (Laurin) | 0.05 |
| 3. α-Amylzimtaldehyd | 1.0 |
| 4. Isoamylsalicylat | 60.0 |
| 5. Anisaldehyd | 30.0 |
| 6. Benzylacetat extra | 20.0 |
| 7. Benzylsalicylat | 80.0 |
| 8. Bergamottöl italienisch | 100.0 |
| 9. Citronellylformiat | 5.0 |
| 10. Zibet Absolue | 1.0 |
| 11. Cumarin rein kristallin | 7.0 |
| 12. Diethylphthalat | 1.8 |
| 13. Dipropylenglycol | 129.9 |
| 14. Geraniol extra | 3.0 |
| 15. Geraniumöl afrikanisch | 70.0 |
| 16. Heliotropin kristallin | 30.0 |
| 17. Hydroxycitronellal | 90.0 |
| 18. Lavendelöl | 50.0 |
| 19. Citronenöl italienisch | 50.0 |
| 20. Limongrasöl rektifiziert | 5.0 |
| 21. Linalool synthetisch | 5.0 |
| 22. Linalylacetat synthetisch | 5.0 |
| 23. Methylanthranilat extra | 3.0 |
| 24. Methyl-β-naphthylketon | 3.0 |
| 25. γ-Undecalacton | 0.1 |
| 26. Petitgrainöl Paraguay rein | 20.0 |
| 27. Phenylacetaldehyd 85 % / PEA | 0.1 |
| 28. Phenylethylalkohol weiss | 15.0 |
| 29. Sandelholzöl ostindisch | 20.0 |
| 30. Estragonöl | 25.0 |
| 31. Terpineol rein | 3.0 |
| 32. Vanillin | 7.0 |
| 33. Vetiveröl (Bourbon) | 10.0 |
| 34. Verbindung **1** | 150.0 |
| | 1000.0 |

### Beispiel 8:

Es wurde eine Herren-Parfümkomposition "Fresh Musk" hergestellt. In Analogie zu Beispiel 7 wurden 10 Gew.-% der Verbindung 1 verwendet. Die Komponenten sind unten aufgeführt.

Die Verbindung **1** verstärkt den Moschuscharakter und fügt eine an Nitromoschus erinnernde Note hinzu. Der zusätzliche Myrrhe-Jasmon-Aspekt mildert die herbkräuterartige Kopfnote ab und gibt ihr dadurch vollkommen neue Facetten. Somit harmoniert die Verbindung **1** sehr gut und synergetisch mit anderen makrocyclischen Moschusriechstoffen, z.B. insbesondere mit Cyclopentadecanolid, und lässt sich gut mit blumigen Noten wie z.B Jasmin verbinden.

### Zusammensetzung:

| Ingredienzien | Gewichts %o |
|---|---|
| 1. Agrudor BAV 645/3 | 15.0 |
| 2. Ambretton (Musk TM II) | 4.0 |
| 3. Benzylacetat extra | 5.0 |
| 4. Benzylsalicylat | 120.0 |
| 5. Cepionat (Hedion) | 5.0 |
| 6. Dipropylenglycol | 480.0 |
| 7. Ethylenbrassylat | 200.0 |
| 8. Hexylzimtaldehyd | 25.0 |
| 9. Lavendelöl (Grosso) | 5.0 |
| 10. Citronenöl italienisch | 5.0 |
| 11. Linalylacetat synthetisch | 25.0 |
| 12. Cyclopentadecanolid | 10.0 |
| 13. Tonkabohnen Resinoid N.1 30 %/DPG | 1.0 |
| 14. Verbindung **1** | 100.0 |
| | 1000.0 |

### Beispiel 9:

Es wurde ein Parfümöl mit moderner Lavendelnote, abgerundet durch feinblumige moschusartige Holztöne, zur Verwendung in Seifen hergestellt, das 10 Gew.-% der Verbindung **1** an Stelle von für diesen Dufttyp sonst üblichem Nitromoschus enthält. Die Komponenten der Komposition sind nachfolgend aufgeführt.
Die Komposition verleiht der Seife einen sehr warm-pudrigen, angenehmen Effekt, der beim Gebrauch derselben den reinigenden und pflegenden Charakter unterstreicht.

### Zusammensetzung:

| Ingredienzien | Gewichts %o |
|---|---|
| 1. Acetaldehydphenethylpropylacetal 10 % DPG | 25.0 |
| 2. Allylphenoxyacetat | 5.0 |
| 3. Aldehyd C10 (n-Decanal) | 6.0 |
| 4. Aldehyd C11(10-Undecenal) 10 % DPG | 10.0 |
| 5. Aldehyd C12 (Laurin) | 10.0 |
| 6. Aldehyd C12 NMA 10 % DPG | 32.0 |
| 7. Allylamylglycolat | 8.0 |
| 8. Capronsäureallylester | 3.0 |
| 9. Bergamylacetat | 25.0 |
| 10. Zimtaldehyd | 2.0 |
| 11. Citronellal | 5.0 |
| 12. Citronellol 750 | 30.0 |
| 13. Cumarin rein kristallin | 5.0 |
| 14. Cyclamenaldehyd extra | 5.0 |
| 15. Dihydromyrcenol | 110.0 |
| 16. Ethylvanillin 10 % DPG | 5.0 |
| 17. Eucalyptol | 5.0 |
| 18. Eugenol rein | 5.0 |
| 19. 3,6-Dimethyl-β-Resocinsäuremethylester | 2.0 |
| 20. Florhydral | 5.0 |
| 21. Fructon | 5.0 |
| 22. Galbanumöl 10 % DPG | 15.0 |
| 23. Geranitril T | 15.0 |
| 24. Geraniumöl afrikanisch | 15.0 |
| 25. α-Hexylzimtaldehyd | 70.0 |
| 26. 2,4,6-Trimethyl-3-cyclohexen-1-carboxaldehyd | 9.0 |
| 27. Lavendelöl (Grosso) | 15.0 |
| 28. Lilial | 120.0 |
| 29. Limettenoxid | 10.0 |
| 30. Linalool synthetisch | 20.0 |
| 31. Menthanylacetat | 25.0 |
| 32. Menthylacetat | 15.0 |
| 33. *para-tert*-Butylcyclohexylacetat | 60.0 |
| 34. Patchouliöl | 18.0 |
| 35. Phenylethylacetat | 10.0 |
| 36. Phenylethylalkohol weiss | 30.0 |
| 37. Rosenoxid CO 10 % DPG | 5.0 |
| 38. Rosmarinöl | 5.0 |
| 39. Krauseminzöl FCC NF extra KPF 10 % DPG | 10.0 |
| 40. Terpineol rein | 50.0 |
| 41. Terpinylacetat | 15.0 |
| 42. Tricylal 10 % DPG | 10.0 |
| 43. Tridecenonitril 10 % DPG | 10.0 |
| 44. Verdol 10% DPG | 15.0 |
| 45. *ortho-tert*-Butylcyclohexylacetat | 10.0 |
| 46. 1-Methyl-4-(4-methyl-3-pentenyl)-3-cyclohexen-1-carboxaldehyd | 15.0 |
| 47. Verbindung **1** | 100.0 |
| | 1000.0 |

Analoge Kompositionen zu den in den Beispielen 7 bis 9 beschriebenen wurden auch mit den Verbindungen **3** und **4** hergestellt.
Aus all diesen beispielhaften Kompositionen geht hervor, dass die erfindungsgemässe Verbindungsklasse, insbesondere mindestens eine der Verbindungen **1, 3** und **4**, vorzugsweise die Verbindung **1**, hervorragend geeignet ist, als Riechstoff eingesetzt zu werden.

## Patentansprüche

1. Verbindungen der allgemeinen Formel **I** 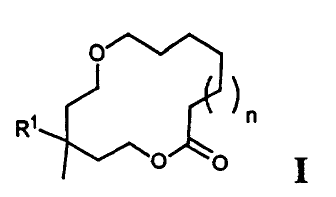 ,worin R¹ = H bei n = 1-4
oder R¹ = CH₃ bei n = 1-3 darstellen.

2. Eine Verbindung nach Anspruch 1 ausgewählt aus der Gruppe
12-Methyl-9-oxa-14-tetradecanolid,
11-Methyl-8-oxa-13-tridecanolid,
13-Methyl-10-oxa-15-pentadecanolid,
14-Methyl-11-oxa-16-hexadecanolid,
12,12-Dimethyl-9-oxa-14-tetradecanolid und
13,13-Dimethyl-10-oxa-15-pentadecanolid.

3. Verwendung mindestens einer der Verbindungen gemäss Anspruch 1 als Riechstoff.

4. Verwendung nach Anspruch 3, **dadurch gekennzeichnet, dass** die Verbindung 12-Methyl-9-oxa-14-tetradecanolid ist.

5. Riechstoffkomposition enthaltend mindestens eine Verbindung der Formel I 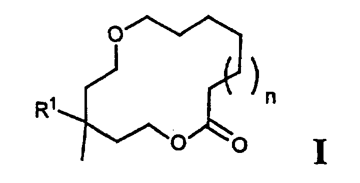 ,worin R¹ = H bei n = 1-4
oder R¹ = CH₃ bei n = 1-3 darstellen.

6. Riechstoffkomposition nach Anspruch 5 enthaltend die Verbindung 12-Methyl-9-oxa-14-tetradecanolid.

7. Riechstoffkomposition nach Anspruch 5 oder 6, **dadurch gekennzeichnet, dass** die Verbindung zu etwa 0.1 bis etwa 25 % darin enthalten ist/sind.

8. Verfahren zur Herstellung einer Verbindung der allgemeinen Formel **I** 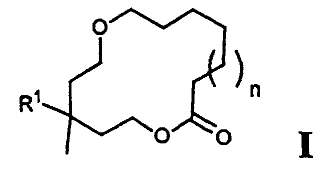 worin R¹ = H bei n = 1-4
oder R¹ = CH₃ bei n = 1-3 darstellt,
**dadurch gekennzeichnet, dass** Hydroxymalonester **10** 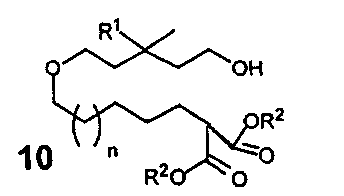 mit n = 1-4 und R² = Alkyl polymerisiert wird.

9. Verfahren nach Anspruch 8, **dadurch gekennzeichnet, dass** die Polymerisierung unter Decarboxylierung erfolgt.

## Claims

1. Compounds of the general formula **I** 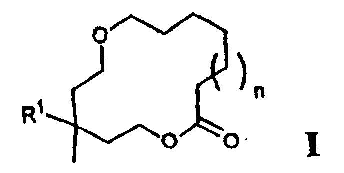 wherein R¹ = H where n = 1-4
or R¹ = CH₃ where n = 1-3.

2. A compound according to claim 1 selected from the group
12-methyl-9-oxa-14-tetradecanolide,
11-methyl-8-oxa-13-tridecanolide,
13-methyl-10-oxa-15-pentadecanolide,
14-methyl-11-oxa-16-hexadecanolide,
12,12-dimethyl-9-oxa-14-tetradecanolide and
13,13-dimethyl-10-oxa-15-pentadecanolide.

3. Use of at least one of the compounds according to claim 1 as an odoriferous substance.

4. Use according to claim 3, **characterised in that** the compound is 12-methyl-9-oxa-14-tetradecanolide.

5. Odoriferous substance composition containing at least one compound of the formula **I** 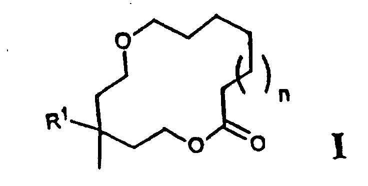 wherein R¹ = H where n = 1-4
or R¹ = CH₃ where n = 1-3.

6. Odoriferous substance composition according to claim 5 containing the compound 12-methyl-9-oxa-14-tetradecanolide.

7. Odoriferous substance composition according to claim 5 or 6, **characterised in that** the compound is contained therein in an amount of about 0.1% to about 25%.

8. Process for the preparation of a compound of the general formula **I** 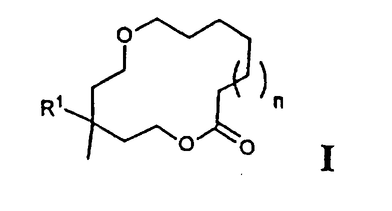 wherein R¹ = H where n = 1-4
or R¹ = CH₃ where n = 1-3.
**characterised in that** hydroxymalonic ester **10** 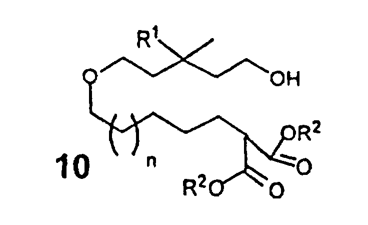 where n = 1-4 and R² = alkyl, is polymerised.

9. Process according to claim 8, **characterised in that** the polymerisation takes place under decarboxylation.

## Revendications

1. Composés de formule générale I 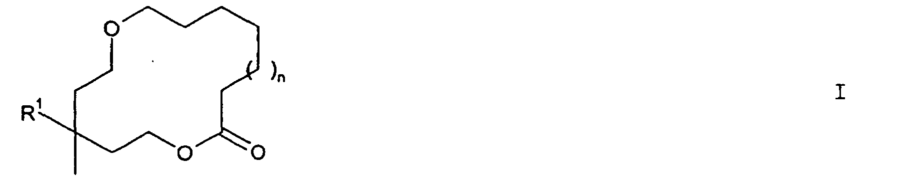 dans laquelle R¹ = H pour n = 1 à 4
ou R¹ = CH₃ pour n = 1 à 3

2. Composé selon la revendication 1, choisi dans l'ensemble :
12-méthyl-9-oxa-14-tétradécanolide,
11-méthyl-8-oxa-13-tridécanolide,
13-méthyl-10-oxa-15-pentadécanolide,
14-méthyl-11-oxa-16-hexadécanolide,
12, 12-diméthyl-9-oxa-14-tétradécanolide, et
13,13-diméthyl-10-oxa-15-pentadécanolide.

3. Utilisation d'au moins l'un des composés selon la revendication 1 en tant que substance odorante.

4. Utilisation selon la revendication 3, **caractérisée en ce que** le composé est le 12-méthyl-9-oxa-14-tétradécanolide.

5. Composition de substances odorantes contenant au moins un composé de Formule I 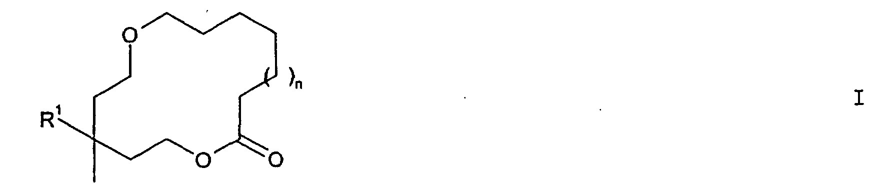 dans laquelle R¹ = H pour n = 1 à 4
ou R¹ = CH₃ pour n = 1 à 3

6. Composition de substances odorantes selon la revendication 5, contenant le composé 12-méthyl-9-oxa-14-tétradécanolide.

7. Compositions de substances odorantes selon la revendication 5 ou 6, **caractérisées en ce que** le ou les composés sont présents en une quantité d'environ 0,1 à environ 25 %.

8. Procédé de préparation d'un composé de formule générale I 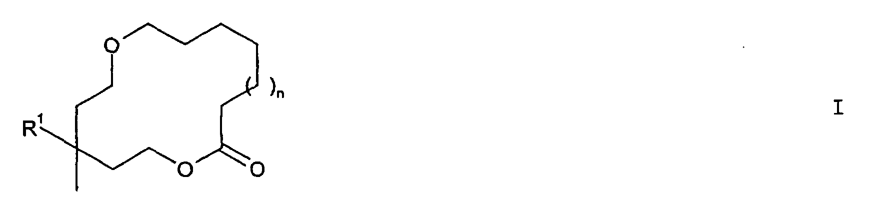 dans laquelle R¹ = H pour n = 1 à 4
ou R¹ = CH₃ pour n = 1 à 3,
**caractérisé en ce qu'**on polymérise l'hydroxymalonate 10 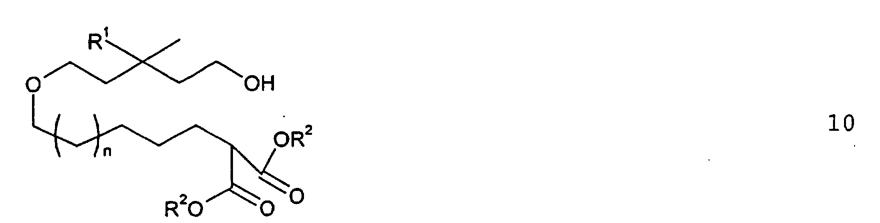 avec n = 1 à 4 et R² est un groupe alkyle.

9. Procédé selon la revendication 8, **caractérisé en ce que** la polymérisation a lieu en présence d'une décarboxylation.
